# EUROPÄISCHE PATENTSCHRIFT

(11) **EP 1 244 615 B1**
(45) Veröffentlichungstag und Bekanntmachung des Hinweises auf die Patenterteilung: **11.08.2004**
(21) Anmeldenummer: 00993607.1
(22) Anmeldetag: 12.12.2000
(51) Int. Cl.: C07C 311/09, C07C 253/14

(54) **VERFAHREN ZUR HERSTELLUNG VON N-SUBSTITUIERTEN 2,4-DIAMINO-5-FLUOR-BENZONITRILEN UND NEUE ZWISCHENPRODUKTE**
METHOD FOR PRODUCING N-SUBSTITUTED 2,4-DIAMINO-5-FLUORO BENZONITRILES AND NOVEL INTERMEDIATES
PROCEDE DE PRODUCTION DE 2,4-DIAMINO-5-FLUOROBENZONITRILES A SUBSTITUTION N, ET NOUVEAUX PRODUITS INTERMEDIAIRES

(30) Priorität: 24.12.1999 DE 19962932
(43) Veröffentlichungstag der Anmeldung: 02.10.2002
(73) Patentinhaber: Bayer CropScience AG, 40789 Monheim (DE)
(72) Erfinder: HUPPERTS, Achim, 40477 Düsseldorf (DE)
(86) Internationale Anmeldenummer: PCT/EP2000/012566
(87) Internationale Veröffentlichungsnummer: WO 2001/047873

(56) Entgegenhaltungen:
- WO-A-95/29158
- WO-A-99/05098

## Beschreibung

Die Erfindung betrifft ein neues Verfahren zur Herstellung von N-substituierten 2,4-Diamino-5-fluor-benzonitrilen, welche als Zwischenprodukte bei der Herstellung von Herbiziden bekannt sind, neue N-substituierte 4-Brom-6-fluor-1,3-phenylendiamine, neue N-substituierte 2-Brom-4-fluor-5-nitro-aniline und neue N-substituierte 2-Brom-4-fluor-aniline als Zwischenprodukte dafür und Verfahren zu deren Herstellung.

Es ist bekannt, daß man bestimmte N-(5-Amino-2-cyano-4-fluor-phenyl)-alkansulfonamide, wie z.B. N-(5-Amino-2-cyano-4-fluor-phenyl)-methansulfonamid, erhält, wenn man entsprechende halogenierte Benzolderivate, wie z.B. 1-Amino-4-cyano-2,5-difluor-benzol, mit Alkansulfonamiden, wie z.B. Methansulfonamid, in Gegenwart eines Säurebindemittels, wie z.B. Kaliumcarbonat, und in Gegenwart eines Verdünnungsmittels, wie z.B. N-Methyl-pyrrolidon, erhitzt (siehe EP-A-648 772). Die gewünschten Produkte werden nach diesem Verfahren jedoch oft in unbefriedigenden Ausbeuten erhalten.

Weiter ist bekannt, daß man bestimmte N-(5-Amino-2-cyano-4-fluor-phenyl)-sulfonamide, wie z.B. N-(5-Amino-2-cyano-4-fluor-phenyl)-methansulfbnamid, erhält, wenn man in einem ersten Schritt 2-Amino-4,5-difluor-benzonitril mit Sulfonsäurehalogeniden, wie z.B. Methansulfonsäurechlorid, in Gegenwart eines Säurebindemittels, wie z.B. Triethylamin, und in Gegenwart eines Verdünnungsmittels, wie z.B. Acetonitril, umsetzt und die hierbei gebildeten Sulfonylierungsprodukte in einem zweiten Schritt mit Ammoniak in Gegenwart eines Verdünnungsmittels, wie z.B. Tetrahydrofuran, umsetzt (vgl. WO-A-99/05098). Auch bei diesem Verfahren werden die gewünschten Produkte oft in unbefriedigenden Ausbeuten erhalten.

Ferner sind bereits einige N-substituierte 4-Chlor-6-fluor-1,3-phenylendiamine aus der (Patent-)Literatur bekannt (vgl. WO-A-98/37065). Entsprechende N-substituierte 4-Brom-6-fluor-1,3-phenylendiamine sind jedoch bisher nicht bekannt geworden.

Es wurde nun gefunden, daß man N-substituierte 2,4-Diamino-5-fluor-benzonitrile der allgemeinen Formel (I) 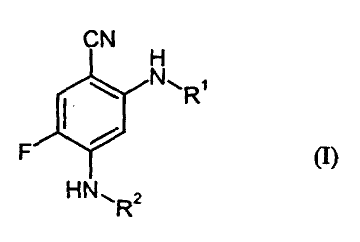 in welcher
- R¹: für jeweils gegebenenfalls substituiertes Alkylsulfonyl oder Arylsulfonyl steht und
- R²: für Wasserstoff oder jeweils gegebenenfalls substituiertes Alkoxycarbonyl oder Aryloxycarbonyl steht,
in hohen Ausbeuten und in sehr guter Qualität erhält, wenn man in einer ersten Umsetzungsstufe 2-Brom-4-fluor-anilin der Formel (II) 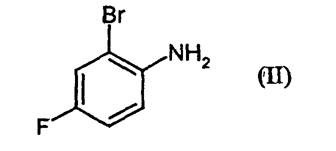 mit einem Acylierungs- oder Sulfonylierungsmittel der allgemeinen Formel (III)

X¹-R¹ (III)

in welcher
- R¹: die oben angegebene Bedeutung hat und
- X¹: für Halogen steht,
gegebenenfalls in Gegenwart eines oder mehrerer Reaktionshilfsmittel und gegebenenfalls in Gegenwart eines oder mehrerer Verdünnungsmittel bei Temperaturen zwischen -20°C und +100°C umsetzt,
die hierbei erhaltenen N-substituierten 2-Brom-4-fluor-aniline der allgemeinen Formel (IV) 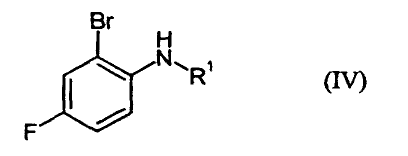 in welcher
- R¹: die oben angegebene Bedeutung hat,
in einer zweiten Umsetzungsstufe mit Acylierungs- oder Sulfonylierungsmitteln der allgemeinen Formel (V)

X²-R³ (V)

in welcher
- R³: für jeweils gegebenenfalls substituiertes Alkylsulfonyl, Alkylcarbonyl, Alkoxycarbonyl, Arylsulfonyl, Arylcarbonyl oder Aryloxycarbonyl steht und
- X²: für Halogen steht,
gegebenenfalls in Gegenwart eines oder mehrerer Reaktionshilfsmittel und gegebenenfalls in Gegenwart eines oder mehrerer Verdünnungsmittel bei Temperaturen zwischen -20°C und 100°C umsetzt, die hierbei erhaltenen N-substituierten 2-Brom-4-fluor-aniline der allgemeinen Formel (VI) 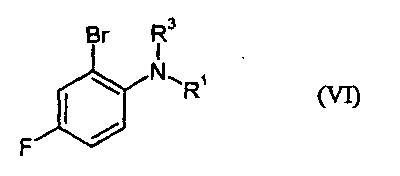 in welcher
- R¹ und R³: die oben angegebene Bedeutung haben,
in einer dritten Umsetzungsstufe mit Nitrierungsmitteln, gegebenenfalls in Gegenwart eines oder mehrerer Nitrierungshilfsmittel und/oder gegebenenfalls in Gegenwart eines oder mehrerer Verdünnungsmittel bei Temperaturen zwischen -30°C und +50°C umsetzt,
die hierbei erhaltenen N-substituierten 2-Brom-4-fluor-5-nitro-aniline der allgemeinen Formel (VII) 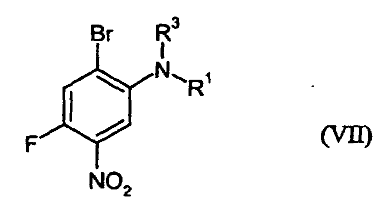 in welcher
- R¹ und R³: die oben angegebene Bedeutung haben,
in einer vierten Umsetzungsstufe mit Reduktionsmitteln, gegebenenfalls in Gegenwart eines oder mehrerer Reduktionshilfsmittel und gegebenenfalls in Gegenwart eines oder mehrerer Verdünnungsmittel, bei Temperaturen zwischen 0°C und 150°C umsetzt, die hierbei erhaltenen N-substituierten 4-Brom-6-fluor-1,3-phenylendiamine der allgemeinen Formel (VIII) 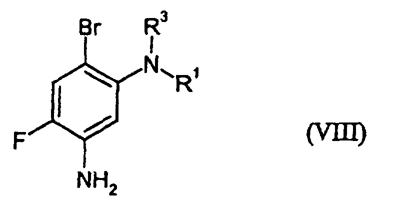 in welcher
- R¹ und R³: die oben angegebene Bedeutung haben,
gegebenenfalls in einer fünften Umsetzungsstufe mit Acylierungsmitteln der allgemeinen Formel (IX)

X³-R²⁻¹ (IX)

in welcher
- R²⁻¹: mit Ausnahme von Wasserstoff die oben für R² angegebene Bedeutung hat und
- X³: für Halogen steht,
gegebenenfalls in Gegenwart eines oder mehrerer Reaktionshilfsmittel und gegebenenfalls in Gegenwart eines oder mehrerer Verdünnungsmittel bei Temperaturen zwischen -20°C und +100°C umsetzt,
die in der vierten bzw. fünften Stufe erhaltenen N-substituierten 4-Brom-6-fluor-1,3-phenylendiamine der allgemeinen Formel (X) 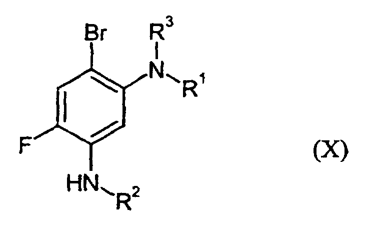 in welcher
- R¹, R² und R³: die oben angegebene Bedeutung haben,
in einer sechsten Stufe mit Wasser in Gegenwart eines oder mehrerer Hydrolysehilfsmittel und gegebenenfalls in Gegenwart eines oder mehrerer Verdünnungsmittel bei Temperaturen zwischen 0°C und 120°C umsetzt,
und die hierbei erhaltenen N-substituierten 4-Brom-6-fluor-1,3-phenylendiamine der allgemeinen Formel (XI) 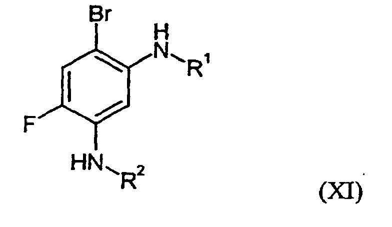 in welcher
- R¹ und R²: die oben angegebene Bedeutung haben,
in einer siebenten Umsetzungsstufe mit einem Metallcyanid gegebenenfalls in Gegenwart eines oder mehrerer Verdünnungsmittel bei Temperaturen zwischen 50°C und 200°C umsetzt.

Überraschenderweise können nach dem erfindungsgemäßen Verfahren die N-substituierten 2,4-Diamino-5-fluor-benzonitrile der allgemeinen Formel (I) auf relativ einfache Weise in hohen Ausbeuten und in sehr guter Qualität erhalten werden.

Dabei sei insbesondere darauf hingewiesen, dass die Reduktion der 2-Brom-4-fluor-5-nitro-aniline der allgemeinen Formel (VII) zu den 4-Brom-6-fluor-1,3-phenylendiaminen der allgemeinen Formel (VIII) sehr gut gelingt, während im allgemeinen die Abspaltung von Brom-Substituenten an Arenen bei der Reduktion von Nitroverbindungen zu entsprechenden Aminoverbindungen eine häufig beobachtete (unerwünschte) Nebenreaktion ist. Da der Austausch des Brom-Substituenten gegen eine Cyanogruppe in der Endstufe besonders glatt verläuft - im Vergleich zur Substitution eines Chloratoms - stellt die erfindungsgemässe Reaktionsführung eine erhebliche Bereicherung des Standes der Technik dar.

Die in der ersten Umsetzungsstufe des erfindungsgemäßen Verfahrens als Ausgangsstoff zu verwendende Verbindung 2-Brom-4-fluor-anilin der Formel (II) ist bereits bekannt (vgl. J. Org. Chem. 28 (1963), 1759-1762).

Die in der ersten Umsetzungsstufe einzusetzenden Acylierungs- oder Sulfonylierungsmittel der allgemeinen Formel (III) sind bekannte Synthesechemikalien.

Bevorzugte Substituenten bzw. bevorzugte Bereiche der oben und nachstehend aufgeführten Formeln vorhandenen Reste werden im Folgenden definiert.
- R¹: steht bevorzugt für gegebenenfalls durch Fluor, Chlor oder Brom substituiertes Alkylsulfonyl mit 1 bis 6 Kohlenstoffatomen oder für gegebenenfalls durch Fluor, Chlor, Brom, C₁-C₄-Alkyl oder C₁-C₄-Alkoxy substituiertes Phenylsulfonyl.
- R²: steht bevorzugt für Wasserstoff, für gegebenenfalls durch Fluor, Chlor oder Brom substituiertes Alkoxycarbonyl mit 1 bis 6 Kohlenstoffatomen in der Alkylgruppe, oder für gegebenenfalls durch Fluor, Chlor, Brom, C₁-C₄-Alkyl oder C₁-C₄-Alkoxy substituiertes Phenoxycarbonyl.
- R³: steht bevorzugt für jeweils gegebenenfalls durch Fluor, Chlor oder Brom substituiertes Alkylsulfonyl, Alkylcarbonyl oder Alkoxycarbonyl mit jeweils 1 bis 6 Kohlenstoffatomen in den Alkylgruppen, oder für jeweils gegebenenfalls durch Fluor, Chlor, Brom, C₁-C₄-Alkyl oder C₁-C₄-Alkoxy substituiertes Phenylsulfonyl, Phenylcarbonyl oder Phenoxycarbonyl.
- X¹: steht bevorzugt für Fluor, Chlor, Brom oder Iod.
- X²: steht bevorzugt für Fluor, Chlor, Brom oder Iod.
- X³: steht bevorzugt für Fluor, Chlor, Brom oder Iod.
- R¹: steht besonders bevorzugt für jeweils gegebenenfalls durch Fluor oder Chlor substituiertes Methylsulfonyl, Ethylsulfonyl, n- oder i-Propylsulfonyl, n-, i-, s- oder t-Butylsulfonyl oder für jeweils gegebenenfalls durch Fluor, Chlor, Brom, Methyl, Ethyl, n- oder i-Propyl, n-, i-, s- oder t-Butyl, Methoxy, Ethoxy, n- oder i-Propoxy substituiertes Phenylsulfonyl.
- R²: steht besonders bevorzugt für Wasserstoff, für jeweils gegebenenfalls durch Fluor oder Chlor substituiertes Methoxycarbonyl, Ethoxycarbonyl, n- oder i-Propoxycarbonyl, n-, i-, s- oder t-Butoxycarbonyl, oder für gegebenenfalls durch Fluor, Chlor, Brom, Methyl, Ethyl, n- oder i-Propyl, n-, i-, s- oder t-Butyl, Methoxy, Ethoxy, n- oder i-Propoxy substituiertes Phenoxycarbonyl.
- R³: steht besonders bevorzugt für jeweils gegebenenfalls durch Fluor oder Chlor substituiertes Methylsulfonyl, Ethylsulfonyl, n- oder i-Propylsulfonyl, n-, i-, s- oder t-Butylsulfonyl, Methylcarbonyl, Ethylcarbonyl, n- oder i-Propylcarbonyl, n-, i-, s- oder t-Butylcarbonyl, Methoxycarbonyl, Ethoxycarbonyl, n- oder i-Propoxycarbonyl, oder für jeweils gegebenenfalls durch Fluor, Chlor, Brom, Methyl, Ethyl, n- oder i-Propyl, n-, i-, s- oder t-Butyl, Methoxy, Ethoxy, n- oder i-Propoxy substituiertes Phenylsulfonyl, Phenylcarbonyl oder Phenoxycarbonyl.
- X¹: steht besonders bevorzugt für Fluor, Chlor oder Brom.
- X²: steht besonders bevorzugt für Fluor, Chlor oder Brom.
- X³: steht besonders bevorzugt für Fluor, Chlor oder Brom.
- R¹: steht ganz besonders bevorzugt für jeweils gegebenenfalls durch Fluor oder Chlor substituiertes Methylsulfonyl, Ethylsulfonyl, n- oder i-Propylsulfonyl, n- oder i-Butylsulfonyl.
- R²: steht ganz besonders bevorzugt für Wasserstoff oder für Methoxycarbonyl, Ethoxycarbonyl, n- oder i-Propoxycarbonyl.
- R³: steht ganz besonders bevorzugt für jeweils gegebenenfalls durch Fluor oder Chlor substituiertes Methylsulfonyl, Ethylsulfonyl, n- oder i-Propylsulfonyl, n- oder i-Butylsulfonyl, Methylcarbonyl, Ethylcarbonyl, n- oder i-Propylcarbonyl, n-, i-, s- oder t-Butylcarbonyl, für Methoxycarbonyl, Ethoxycarbonyl, n- oder i-Propoxycarbonyl.
- X¹: steht ganz besonders bevorzugt für Chlor.
- X²: steht ganz besonders bevorzugt für Chlor.
- X³: steht ganz besonders bevorzugt für Chlor.

Die Ausgangsstoffe der allgemeinen Formeln (III), (V) und (IX) sind bekannte Synthesechemikalien.

Die erste und die zweite Umsetzungsstufe des erfindungsgemäßen Verfahrens werden vorzugsweise in Gegenwart eines oder mehrerer Reaktionshilfsmittel durchgeführt. Als Reaktionshilfsmittel kommen hierbei im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -noder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2.2.2]-octan (DABCO), 1,5-Diazabicyclo[4.3.0]-non-5-en (DBN), oder 1,8-Diazabicyclo[5.4.0]-undec-7-en (DBU).

Vorzugsweise werden basische organische Stickstoffverbindungen, insbesondere Pyridin oder Triethylamin, als Reaktionshilfsmittel eingesetzt.

Die erste und die zweite Umsetzungsstufe des erfindungsgemäßen Verfahrens werden vorzugsweise in Gegenwart eines oder mehrerer Verdünnungsmittel durchgeführt. Als Verdünnungsmittel kommen hierbei vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Vorzugsweise werden halogenierte Kohlenwasserstoffe, insbesondere Methylenchlorid, oder Nitrile, insbesondere Acetonitril, als Verdünnungsmittel eingesetzt.

Die Reaktionstemperaturen können bei der Durchführung der ersten und der zweiten Umsetzungsstufe des erfindungsgemäßen Verfahrens in einem größeren Bereich variiert werden. Im allgemeinen arbeitet man bei Temperaturen zwischen -20°C und +100°C, vorzugsweise zwischen -10°C und +80°C, insbesondere zwischen 0°C und +60°C.

Die erste und die zweite Umsetzungsstufe des erfindungsgemäßen Verfahrens werden im allgemeinen unter Normaldruck durchgeführt. Es ist jedoch auch möglich, diese Umsetzungen unter erhöhtem oder vermindertem Druck - im allgemeinen zwischen 0,1 bar und 10 bar - durchzuführen.

Zur Durchführung der ersten Umsetzungsstufe des erfindungsgemäßen Verfahrens setzt man auf 1 Mol 2-Brom-4-fluor-anilin der Formel (II) im allgemeinen zwischen 1 Mol und 5 Mol, vorzugsweise zwischen 1,0 Mol und 2,5 Mol Acylierungs- oder Sulfonylierungsmittel der allgemeinen Formel (III) und zwischen 1 und 5 Mol, vorzugsweise zwischen 1,0 Mol und 2,5 Mol Reaktionshilfsmittel ein.

In einer bevorzugten Ausführungsform der ersten Umsetzungsstufe wird das 2-Brom-4-fluor-anilin der Formel (II) zusammen mit einem Reaktionshilfsmittel in einem Verdünnungsmittel vorgelegt und das Acylierungs- oder Sulfonylierungsmittel der allgemeinen Formel (III) wird dann unter Rühren (gegebenenfalls unter leichtem Kühlen) langsam in diese Mischung eindosiert. Die komplette Reaktionsmischung wird dann (gegebenenfalls bei etwas erhöhter Temperatur) bis zum Ende der Umsetzung gerührt.

Zur Durchführung der zweiten Umsetzungsstufe des erfindungsgemäßen Verfahrens setzt man auf 1 Mol eines N-substituierten 2-Brom-4-fluor-anilins der allgemeinen Formel (IV) im allgemeinen zwischen 1 Mol und 3 Mol, vorzugsweise zwischen 1,0 Mol und 1,5 Mol Acylierungs- oder Sulfonylierungsmittel der allgemeinen Formel (V) und zwischen 1 und 3 Mol, vorzugsweise zwischen 1,0 Mol und 1,5 Mol Reaktionshilfsmittel ein.

In einer bevorzugten Ausführungsform der zweiten Umsetzungsstufe wird das N-substituierte 2-Brom-4-fluor-anilin der Formel (IV) zusammen mit einem Reaktionshilfsmittel in einem Verdünnungsmittel vorgelegt und das Acylierungs- oder Sulfonylierungsmittel der allgemeinen Formel (V) wird dann unter Rühren (gegebenenfalls unter leichtem Kühlen) langsam in diese Mischung eindosiert. Die komplette Reaktionsmischung wird dann (gegebenenfalls bei etwas erhöhter Temperatur) bis zum Ende der Umsetzung gerührt.

Sofern in Einzelfällen die Acylierungs- oder Sulfonylierungsmittel der ersten und der zweiten Umsetzungsstufe identisch sind, können die beiden Stufen zu einer Umsetzungsstufe zusammengefasst werden, d.h. sie werden in einem Schritt durchgeführt.

Die Aufarbeitung der Produkte der ersten wie auch der zweiten Umsetzungsstufe kann auf übliche Weise durchgeführt werden. Sofern das eingesetzte Verdünnungsmittel mit Wasser praktisch nicht mischbar ist, kann direkt mit Wasser gewaschen werden. Andernfalls wird (gegebenenfalls nach Einengen) mit Wasser und einem mit Wasser praktisch nicht mischbaren organischen Lösungsmittel, wie z.B. Methylenchlorid geschüttelt. Aus der organischen Phase kann man dann, gegebenenfalls nach Trocknen und Filtrieren, durch sorgfältiges Abdestillieren des Lösungsmittels unter vermindertem Druck das Intermediat der Formel (IV) bzw. der Formel (VI) als Rückstand erhalten. In manchen Fällen fallen die Produkte der Formeln (IV) bzw. (VI) auch kristallin an, wenn man die Reaktionsmischung in wässrige Säure (z.B. Salzsäure, gegebenenfalls mit Eis versetzt) gießt. Sie können dann durch Absaugen isoliert werden.

Die nach der ersten Umsetzungsstufe des erfindungsgemäßen Verfahrens erhältlichen N-substituierten 2-Brom-4-fluor-aniline der allgemeinen Formel (IV) sind mit Ausnahme der Verbindungen N-(2-Brom-4-fluor-phenyl)-benzolsulfonamid (vgl. Izv. Akad. Nauk SSSR, Ser. Khim. (1985), (4), 900-904 - zitiert in Chem. Abstracts 103:141325) und N-(2-Brom-4-fluor-phenyl)-1,1,1-trifluor-methansulfonamid (vgl. J. Org. Chem. (1975), 40 (4), 428-431; US-A-3920444) noch nicht aus der Literatur bekannt; sie sind unter Ausnahme von N-(2-Brom-4-fluor-phenyl)-benzolsulfonamid und N-(2-Brom-4-fluor-phenyl)-1,1,1-trifluor-methansulfonamid als neue Stoffe auch Gegenstand der vorliegenden Anmeldung.

Die nach der zweiten Umsetzungsstufe des erfindungsgemäßen Verfahrens erhältlichen N-substituierten 2-Brom-4-fluor-aniline der allgemeinen Formel (VI) sind noch nicht aus der Literatur bekannt; sie sind als neue Stoffe auch Gegenstand der vorliegenden Anmeldung.

Die dritte Umsetzungsstufe des erfindungsgemäßen Verfahrens wird unter Verwendung eines Nitrierungsmittels durchgeführt. Als Nitrierungsmittel wird vorzugsweise Salpetersäure (welche gegebenenfalls bis zu 30% Wasser enthält) eingesetzt.

Die dritte Umsetzungsstufe des erfindungsgemäßen Verfahrens wird gegebenenfalls unter Verwendung eines oder mehrerer Nitrierungshilfsmittel durchgeführt. Als Nitrierungshilfsmittel kommen vorzugsweise (praktisch wasserfreie) Säuren oder Säureanhydride, wie z.B. Schwefelsäure, "Oleum", Essigsäure oder Acetanhydrid, in Betracht.

Gemische aus konz. Salpetersäure und konz. Schwefelsäure werden als Gemische aus Nitrierungsmittel und Nitrierungshilfsmittel ganz besonders bevorzugt.

Die dritte Umsetzungsstufe des erfindungsgemäßen Verfahrens wird gegebenenfalls in Gegenwart eines oder mehrerer Verdünnungsmittel durchgeführt. Als Verdünnungsmittel sind vor allem inerte organische Lösungsmittel, insbesondere halogenierte Alkane, wie z.B. Methylenchlorid, Chloroform oder Tetrachlormethan, geeignet.

Die Reaktionstemperaturen können bei der Durchführung der dritten Umsetzungsstufe des erfindungsgemäßen Verfahrens in einem größeren Bereich variiert werden. Im allgemeinen arbeitet man bei Temperaturen zwischen -30°C und +50°C, vorzugsweise zwischen -20°C und +40°C, insbesondere zwischen -10°C und +30°C.

Die dritte Umsetzungsstufe des erfindungsgemäßen Verfahrens wird im allgemeinen unter Normaldruck durchgeführt. Es ist jedoch auch möglich, die dritte Stufe des erfindungsgemäßen Verfahrens unter erhöhtem oder vermindertem Druck - im allgemeinen zwischen 0,1 bar und 10 bar - durchzuführen.

Zur Durchführung der dritten Umsetzungsstufe des erfindungsgemäßen Verfahrens setzt man auf 1 Mol eines N-substituierten 2-Brom-4-fluor-anilins der allgemeinen Formel (VI) im allgemeinen zwischen 1 Mol und 20 Mol, vorzugsweise zwischen 2 und 10 Mol Nitrierungsmittel, und zwischen 1 und 50 Mol, vorzugsweise zwischen 2 und 30 Mol Nitrierungshilfsmittel ein.

In einer bevorzugten Ausführungsform der dritten Umsetzungsstufe wird das Nitrierungsmittel - vorzugsweise zusammen mit einem Nitrierungshilfsmittel - vorgelegt und das N-substituierte 2-Brom-4-fluor-anilin wird - vorzugsweise unter Kühlen - zudosiert. Die Reaktionsmischung wird bis zum Ende der Umsetzung gerührt.

Die Aufarbeitung der Produkte der dritten Umsetzungsstufe kann auf übliche Weise durchgeführt werden. Vorzugsweise wird die Reaktionsmischung auf Eis gegossen, das kristalline Produkt durch Absaugen isoliert, mit Wasser und einem mit Wasser praktisch nicht mischbaren organischen Lösungsmittel, wie z.B. Essigsäureethylester, geschüttelt, die organische Phase mit gesättigter wässriger Natriumbicarbonat-Lösung gewaschen, mit Natriumsulfat getrocknet und filtriert. Durch sorgfältiges Abdestillieren des Lösungsmittels unter vermindertem Druck wird das N-substituierte 2-Brom-4-fluor-5-nitro-anilin der Formel (VII) als Rückstand erhalten.

Die nach der dritten Umsetzungsstufe des erfindungsgemäßen Verfahrens erhältlichen N-substituierten 2-Brom-4-fluor-5-nitro-aniline der allgemeinen Formel (VII) sind noch nicht aus der Literatur bekannt; sie sind als neue Stoffe auch Gegenstand der vorliegenden Anmeldung.

Die vierte Umsetzungsstufe des erfindungsgemäßen Verfahrens wird unter Verwendung eines Reduktionsmittels gegebenenfalls in Gegenwart eines oder mehrerer Reduktionshilfsmittel und/oder gegebenenfalls in Gegenwart eines oder mehrerer Verdünnungsmittel durchgeführt. Vorzugsweise werden hierbei die für die Umwandlung von aromatischen Nitroverbindungen in entsprechende Aminoverbindungen üblichen Reduktionsmittel (gegebenenfalls zusammen mit geeigneten Reduktionshilfsmitteln und Verdünnungsmitteln) eingesetzt. Hierzu gehören vorzugsweise (a) Wasserstoff in Gegenwart eines Katalysators wie z.B. Platin oder Palladium (jeweils gegebenenfalls "vergiftet" und auf einem Trägermaterial wie z.B. Aktivkohle oder Bariumsulfat, Raney-Nickel oder Raney-Cobalt) und in Gegenwart eines Verdünnungsmittels wie z.B. Tetrahydrofuran oder Dioxan, (b) Metalle oder Metallsalze, wie z.B. Zinn, Zinn(II)-chlorid, Eisen (-Pulver) in Gegenwart einer Säure wie z.B. Salzsäure oder Essigsäure.

Die Reaktionstemperaturen können bei der Durchführung der vierten Umsetzungsstufe des erfindungsgemäßen Verfahrens in einem größeren Bereich variiert werden. Im allgemeinen arbeitet man bei Temperaturen zwischen 0°C und 150°C, vorzugsweise zwischen 10°C und 120°C, insbesondere zwischen 20°C und 100°C.

Zur Durchführung der vierten Umsetzungsstufe des erfindungsgemäßen Verfahrens setzt man auf 1 Mol eines N-substituierten 2-Brom-4-fluor-5-nitro-anilins der allgemeinen Formel (VII) im allgemeinen zwischen 1 Mol und 20 Mol, vorzugsweise zwischen 2 und 10 Mol Reduktionsmittel ein.

In einer bevorzugten Ausführungsform der vierten Umsetzungsstufe wird das N-substituierte 2-Brom-4-fluor-5-nitro-anilin - vorzugsweise zusammen mit einem Reduktionshilfsmittel - vorgelegt und das Reduktionsmittel wird zudosiert. Die Reaktionsmischung wird dann bis zum Ende der Umsetzung gerührt.

Die Aufarbeitung der Produkte der vierten Umsetzungsstufe kann auf übliche Weise durchgeführt werden. Vorzugsweise wird die Reaktionsmischung filtriert und das Filtrat unter vermindertem Druck eingeengt. Der Rückstand wird in einem mit Wasser nicht mischbaren organischen Lösungsmittel wie z.B. Essigsäureethylester aufgenommen, mit wässrigen Natriumbicarbonat-Lösung gewaschen, über Natriumsulfat getrocknet und filtriert. Durch Abdestillieren des Lösungsmittels vom Filtrat unter vermindertem Druck sorgfältig wird das N-substituierte 4-Brom-6-fluor-1,3-phenylendiamin der allgemeinen Formel (VIII) als Rückstand erhalten.

Die nach der vierten Umsetzungsstufe des erfindungsgemäßen Verfahrens erhältlichen N-substituierten 2-Brom-6-fluor-1,3-phenylendiamine der allgemeinen Formel (VIII) sind noch nicht aus der Literatur bekannt; sie sind als neue Stoffe auch Gegenstand der vorliegenden Anmeldung.

Die fünfte Umsetzungsstufe des erfindungsgemäßen Verfahrens wird vorzugsweise in Gegenwart eines oder mehrerer Reaktionshilfsmittel durchgeführt. Als Reaktionshilfsmittel kommen hierbei im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, - hydride, -hydroxide oder -alkanolate, wie beispielsweise Natrium-, Kalium- oder Calcium-acetat, Lithium-, Natrium-, Kalium- oder Calcium-amid, Natrium-, Kaliumoder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2.2.2]-octan (DABCO), 1,5-Diazabicyclo-[4.3.0]-non-5-en (DBN), oder 1,8-Diazabicyclo[5.4.0]-undec-7-en (DBU).

Vorzugsweise werden basische organische Stickstoffverbindungen, insbesondere Pyridin oder Triethylamin, als Reaktionshilfsmittel eingesetzt.

Die fünfte Umsetzungsstufe des erfindungsgemäßen Verfahrens wird vorzugsweise in Gegenwart eines oder mehrerer Verdünnungsmittel durchgeführt. Als Verdünnungsmittel kommen hierbei vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether, Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Vorzugsweise werden aprotisch polare Lösungsmittel aus der Reihe der Ketone wie z.B. Aceton, Butanon oder Methyl-isobutyl-keton, der Nitrile wie z.B. Acetonitril, Propionitril oder Butyronitril, oder der Amide wie z.B. N,N-Dimethyl-formamid oder N,N-Dimethyl-acetamid, eingesetzt.

Die Reaktionstemperaturen können bei der Durchführung der fünften Umsetzungsstufe des erfindungsgemäßen Verfahrens in einem größeren Bereich variiert werden. Im allgemeinen arbeitet man bei Temperaturen zwischen -20°C und +100°C, vorzugsweise zwischen -10°C und 80°C, insbesondere zwischen 0°C und 60°C.

Die fünfte Umsetzungsstufe des erfindungsgemäßen Verfahrens wird im allgemeinen unter Normaldruck durchgeführt. Es ist jedoch auch möglich, die fünfte Umsetzungsstufe unter erhöhtem oder vermindertem Druck - im allgemeinen zwischen 0,1 bar und 10 bar - durchzuführen.

Zur Durchführung der fünften Umsetzungsstufe des erfindungsgemäßen Verfahrens setzt man auf 1 Mol eines N-substituierten 4-Brom-6-fluor-1,3-phenylendiamins der allgemeinen Formel (VIII) im allgemeinen zwischen 0,9 Mol und 1,5 Mol, vorzugsweise zwischen 0,95 Mol und 1,2 Mol eines Acylierungsmittels der allgemeinen Formel (IX) und zwischen 1,0 und 2,0 Mol, vorzugsweise zwischen 1,1 und 1,5 Mol eines Reaktionshilfsmittels ein.

In einer bevorzugten Ausführungsform der fünften Umsetzungsstufe des erfindungsgemäßen Verfahrens wird das 4-Brom-6-fluor-1,3 phenylendiamin der allgemeinen Formel (VIII) mit einem Reaktionshilfsmittel in einem Verdünnungsmittel vorgelegt und ein Acylierungsmittel der allgemeinen Formel (IX) wird - gegebenenfalls unter leichtem Kühlen - zudosiert. Die Reaktionsmischung wird bis zum Ende der Umsetzung gerührt.

Die Aufarbeitung der Produkte der fünften Umsetzungsstufe kann auf übliche Weise durchgeführt werden. Beispielsweise kann die Reaktionsmischung auf (Eis-) Wasser gegeben werden und das Produkt - sofern es kristallin anfällt - durch Absaugen isoliert werden. Es kann aber auch mit Wasser und einem mit Wasser praktisch nicht mischbaren organischen Lösungsmittel wie z.B. Methylenchlorid geschüttelt, die organische Phase mit Wasser gewaschen, getrocknet und filtriert werden. Durch sorgfältiges Abdestillieren des Lösungsmittel unter vermindertem Druck kann das N-substituierte 4-Brom-6-fluor-1,3-phenylendiamin der allgemeinen Formel (X) als Rückstand erhalten werden.

Die sechste Umsetzungsstufe des erfindungsgemäßen Verfahrens wird in Gegenwart eines oder mehrerer Hydrolysehilfsmittel durchgeführt. Als Hydrolysehilfsmittel kommen hierbei vorzugsweise anorganische Basen in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -noder -i-propanolat, -n-, -i-, -s- oder -t-butanolat.

Vorzugsweise werden Alkalimetall- oder Erdalkalimetall-hydride oder -hydroxide, insbesondere Natriumhydrid sowie Natrium- oder Kalium-hydroxid als Hydrolysehilfsmittel eingesetzt.

Die sechste Umsetzungsstufe des erfindungsgemäßen Verfahrens wird gegebenenfalls unter Verwendung eines oder mehrerer Verdünnungsmittel durchgeführt. Als Verdünnungsmittel kommen hierbei neben Wasser vor allem polare organische Lösungsmittel in Betracht. Hierzu gehören insbesondere Ether, wie Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Sulfoxide, wie Dimethylsulfoxid; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung der sechsten Umsetzungsstufe des erfindungsgemäßen Verfahrens in einem größeren Bereich variiert werden. Im allgemeinen arbeitet man bei Temperaturen zwischen -10°C und +120°C, vorzugsweise zwischen 0°C und 100°C.

In einer bevorzugten Ausführungsform der sechsten Umsetzungsstufe des erfindungsgemäßen Verfahrens wird ein N-substituiertes 4-Brom-6-fluor-1,3-phenylendiamin der allgemeinen Formel (X) mit Wasser und einem Reaktionshilfsmittel - und gegebenenfalls einem weiteren Verdünnungsmittel - vermischt und die Reaktionsmischung wird bis zum Ende der Umsetzung gerührt.

Die Aufarbeitung der Produkte der sechsten Umsetzungsstufe kann auf übliche Weise durchgeführt werden. Beispielsweise kann die Reaktionsmischung auf (Eis-) Wasser gegeben werden und nach Ansäuern mit einer starken Säure wie z.B. Salzsäure das N-substituierte 4-Brom-6-fluor-1,3-phenylendiamin der allgemeinen Formel (XI) als kristallines Produkt durch Absaugen isoliert werden.

Die nach der sechsten Umsetzungsstufe des erfindungsgemäßen Verfahrens erhältlichen N-substituierten 4-Brom-6-fluor-1,3-phenylendiamine der allgemeinen Formel (XI) sind noch nicht aus der Literatur bekannt; sie sind als neue Stoffe auch Gegenstand der vorliegenden Anmeldung.

Die siebente Umsetzungsstufe des erfindungsgemäßen Verfahrens wird unter Verwendung eines Metallcyanids durchgeführt. Als Metallcyanide sind hierbei Alkalimetall- oder Erdalkalimetall-cyanide wie z.B. Natrium-, Kalium-, Rubidium-, Cäsium-, Magnesium-, Calcium- und Barium-cyanid, insbesondere aber Übergangsmetallcyanide wie Kupfer(I)-cyanid zu nennen. Auch Gemische der genannten Metallcyanide, in denen jedoch in jedem Fall Kupfer(I)-cyanid enthalten sein sollte, können eingesetzt werden.

Die siebente Umsetzungsstufe des erfindungsgemäßen Verfahrens wird gegebenenfalls unter Verwendung eines oder mehrerer Verdünnungsmittel durchgeführt. Als Verdünnungsmittel kommen hierbei vor allem aprotisch-polare organische Lösungsmittel in Betracht. Hierzu gehören insbesondere Ether, wie Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Vorzugsweise wird ein hochsiedendes aprotisch-polares organisches Lösungsmittel wie z.B. N-Methyl-pyrrolidon als Verdünnungsmittel eingesetzt.

Die Reaktionstemperaturen können bei der Durchführung der siebenten Umsetzungsstufe des erfindungsgemäßen Verfahrens in einem größeren Bereich variiert werden. Im allgemeinen arbeitet man bei Temperaturen zwischen 50°C und 200°C, vorzugsweise zwischen 80°C und 180°C, insbesondere zwischen 100°C und 160°C.

Die siebente Umsetzungsstufe des erfindungsgemäßen Verfahrens wird im allgemeinen unter Normaldruck durchgeführt. Es ist jedoch auch möglich, die siebente Stufe unter erhöhtem oder vermindertem Druck - im allgemeinen zwischen 0,1 bar und 10 bar - durchzuführen.

Zur Durchführung der siebenten Umsetzungsstufe des erfindungsgemäßen Verfahrens setzt man auf 1 Mol eines N-substituierten 4-Brom-6-fluor-1,3-phenylendiamins der allgemeinen Formel (XI) im allgemeinen zwischen 1 Mol und 1,5 Mol, vorzugsweise zwischen 1,1 und 1,3 Mol eines Metallcyanids ein.

In einer bevorzugten Ausführungsform der siebenten Umsetzungsstufe des erfindungsgemäßen Verfahrens werden das N-substituierte 4-Brom-6-fluor-1,3-phenylendiamin der allgemeinen Formel (XI), das Metallcyanid und das Verdünnungsmittel bei Raumtemperatur vermischt und bei erhöhter Temperatur bis zum Ende der Umsetzung gerührt.

Die Aufarbeitung der Produkte der siebenten Umsetzungsstufe kann auf übliche Weise durchgeführt werden. Beispielsweise wird unter vermindertem Druck eingeengt, der Rückstand mit Essigsäureethylester und salzsaurer wässriger Eisen(III)-chlorid-Lösung verrührt, und über Kieselgel filtriert. Die organische Phase wird dann abgetrennt, mit Natriumsulfat getrocknet und filtriert. Nach Abdestillieren des Lösungsmittel unter vermindertem Druck kann das N-substituierte 2,4-Diamino-5-fluor-benzonitril der allgemeinen Formel (I) als Rückstand erhalten werden.

Die nach dem erfindungsgemäßen Verfahren herzustellenden Verbindungen der allgemeinen Formel (I) können als Zwischenprodukte zur Herstellung von herbizid wirksamen Verbindungen verwendet werden (vgl. EP-A-648749, EP-A-648772, WO-A-95/29158).

Die Zwischenprodukte der allgemeinen Formeln (VIII), (X) und (XI) können ebenfalls als Vorstufen für die Herstellung von Herbiziden eingesetzt werden (vgl. EP-A-563384).

### Herstellungsbeispiele:

### Erste Umsetzungsstufe:

### Beispiel (IV-1)

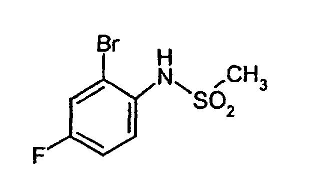

In einem 250 ml Dreihalskolben mit Innenthermometer, Tropftrichter und Rührer werden 18,5 g (0,097 mol) 2-Brom-4-fluor-anilin in 100 ml Methylenchlorid und 10 g (0,12 mol) Pyridin gelöst und bei 5°C mit 12,6 g (0,11 mol) Methansulfonsäurechlorid versetzt. Die Lösung wird 60 Minuten bei 20°C nachgerührt und dann mit 50 ml Wasser versetzt. Die Phasen werden getrennt und die wäßrige Phase wird mit 50 ml Methylenchlorid extrahiert. Die vereinigten organischen Phasen werden mit 50 ml Wasser gewaschen und über Natriumsulfat getrocknet. Nach Entfernen der Lösungsmittel am Rotationsverdampfer erhält man 21,9 g reines N-(2-Brom-4-fluorphenyl)-methansulfonsäureamid (84 % der Theorie).

### Beispiel (IV-2)

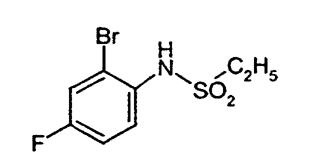

In einem 1 l Dreihalskolben mit Innenthermometer, Tropftrichter und Rührer werden 95 g (0,5 mol) 2-Brom-4-fluor-anilin in 400 ml Methylenchlorid und 44 g (0,55 mol) Pyridin gelöst und bei 0°C bis 5°C mit 65 g (0,5 mol) Ethansulfonsäurechlorid versetzt. Die Lösung wird 60 Minuten im Eisbad und 3 Stunden bei 20°C nachgerührt und dann mit 250 ml Wasser versetzt. Die Phasen werden getrennt und die wäßrige Phase wird mit 100 ml Methylenchlorid extrahiert. Die vereinigten organischen Phasen werden mit 100 ml Wasser gewaschen und über Natriumsulfat getrocknet. Nach Entfernen der Lösungsmittel am Rotationsverdampfer erhält man 135 g N-(2-Brom-4-fluor-phenyl)-ethansulfonsäureamid (91 % der Theorie), das laut gaschromatografischer Analyse 95,8 %ig ist (Schmelzpunkt: 79-81°C).

### Erste und zweite Umsetzungsstufe:

### Beispiel (VI-1)

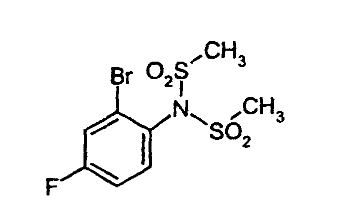

In einem 250 ml Dreihalskolben mit Innenthermometer, Tropftrichter und Rührer werden 19,0 g (0,1 mol) 2-Brom-4-fluor-anilin in 100 ml Acetonitril und 22,2 g (0,22 mol) Triethylamin gelöst und bei 10°C bis 20°C mit 25,2 g (0,22 mol) Methansulfonsäurechlorid versetzt. Die Lösung wird 60 Minuten bei 20°C nachgerührt und dann auf ein Gemisch aus 200 ml Eiswasser und 20 ml konz. Salzsäure gegossen. Der ausgefallene Feststoff wird abgesaugt, in 50 ml Methylenchlorid gelöst und die organische Phase wird über Natriumsulfat getrocknet. Nach Entfernen des Lösungsmittels am Rotationsverdampfer erhält man 30,0 g N-(2-Brom-4-fluor-phenyl)-N-methylsulfonyl-methansulfonsäureamid (87 % der Theorie), das laut HPLC 97,1 %ig ist.

### Beispiel (VI-2)

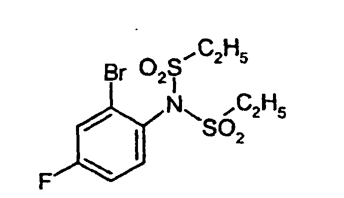

In einem 250 ml Dreihalskolben mit Innenthermometer, Tropftrichter und Rührer werden 28,2 g (0,1 mol) N-(2-Brom-4-fluor-phenyl)-ethansulfonsäureamid in 100 ml Acetonitril und 11 g (0,11 mol) Triethylamin gelöst und bei 15°C bis 20°C mit 13 g (0,1 mol) Ethansulfonsäurechlorid versetzt. Die Lösung wird 60 Minuten bei 20°C nachgerührt und dann auf ein Gemisch aus 200 ml Eiswasser und 20 ml konz. Salzsäure gegossen. Der ausfallende Feststoff wird abgesaugt, in 50 ml Methylenchlorid gelöst und die organische Phase über Natriumsulfat getrocknet. Nach Entfernen des Lösungsmittel am Rotationsverdampfer erhält man 31,6 g N-(2-Brom-4-fluor-phenyl)-N-ethylsulfonyl-ethansulfonsäureamid (81 % der Theorie), das laut HPLC 95,5 %ig ist (Schmelzpunkt: 114-116°C).

### Zweite Umsetzungsstufe:

### Beispiel (VI-3)

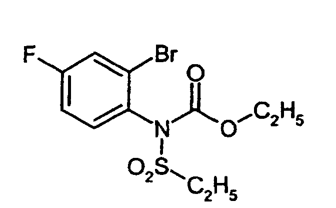

In einem 500 ml Dreihalskolben mit Innenthermometer, Tropftrichter und Rührer werden 56,2g (0,199mol) N-(2-Brom-4-fluor-phenyl)-ethansulfonsäureamid in 200 ml Methylenchlorid und 32 g (0,4 mol) Pyridin gelöst und bei 0°C bis 5°C mit 23,9 g (0,22 mol) Chlorameisensäure-ethylester versetzt. Die Lösung wird 2 Stunden bei 20°C nachgerührt und dann mit 100 ml Wasser versetzt. Die Phasen werden getrennt und die wäßrige Phase wird mit 50 ml Methylenchlorid extrahiert. Die vereinigten organischen Phasen werden mit 100 ml Wasser gewaschen und über Natriumsulfat getrocknet. Nach Entfernen des Lösungsmittels am Rotationsverdampfer erhält man 65,8 g N-(2-Brom-4-fluor-phenyl)-N-ethoxycarbonyl-ethansulfonsäureamid (90,5 % der Theorie), das laut GC 96,9 %ig ist.

### Dritte Umsetzungsstufe:

### Beispiel (VII-1)

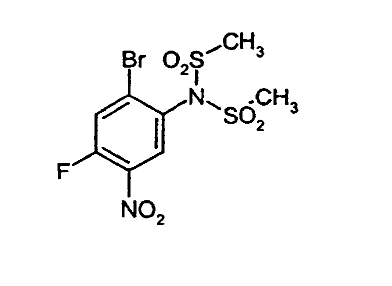

In einem 250 ml Zweihalskolben mit Innenthermometer und Rührer werden 100 ml konz. Schwefelsäure und 35 ml konz. Salpetersäure vorgelegt und auf 0°C bis 5°C gekühlt. Zu diesem Gemisch werden 24,3 g (0,07 mol) N-(2-Brom-4-fluor-phenyl)-N-methylsulfonyl-methansulfonsäureamid gegeben. Man läßt den Ansatz auf 20°C erwärmen, rührt eine Stunde nach und gibt die Reaktionslösung dann auf 300 g Eis. Der ausfallende Feststoff wird abgesaugt und in 100 ml Essigsäureethylester aufgenommen. Die organische Phase wird mit 50 ml Wasser und dann mit 50 ml gesättigter Natriumbicarbonat-Lösung gewaschen und über Natriumsulfat getrocknet. Nach Entfernen des Lösungsmittels am Rotationsverdampfer erhält man 26,5 g reines N-(2-Brom-4-fluor-5-nitro-phenyl)-N-methylsulfonyl-methansulfonsäureamid (97 % der Theorie).

### Beispiel (VII-2)

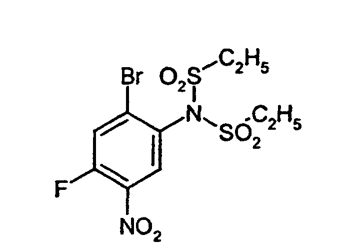

In einem 250 ml Zweihalskolben mit Innenthermometer und Rührer werden 100 ml konz. Schwefelsäure und 35 ml konz. Salpetersäure vorgelegt und auf 0°C bis 5°C gekühlt. Zu diesem Gemisch werden 29,9 g (0,08 mol) N-(2-Brom-4-fluor-phenyl)-N-ethylsulfonyl-ethansulfonsäureamid gegeben. Man läßt den Ansatz auf 20°C erwärmen, rührt eine Stunde nach und gibt die Reaktionslösung dann auf 300 g Eis. Der ausfallende Feststoff wird abgesaugt und in 100 ml Essigsäureethylester aufgenommen. Die organische Phase wird mit 50 ml Wasser und dann mit 50 ml gesättigter Natriumbicarbonat-Lösung gewaschen und über Natriumsulfat getrocknet. Nach Entfernen des Lösungsmittels am Rotationsverdampfer erhält man 29, 1 g N-(2-Brom-4-fluor-5-nitro-phenyl)-N-ethylsulfonyl-ethansulfonsäureamid (81,5 % der Theorie), das laut HPLC 93,9 %ig ist (Schmelzpunkt: 144-146°C).

### Beispiel (VII-3)

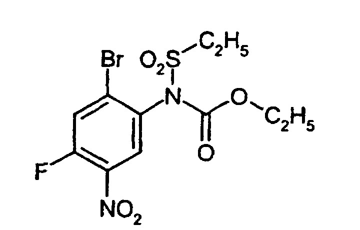

In einem 500 ml Zweihalskolben mit Innenthermometer und Rührer werden 270 ml konz. Schwefelsäure und 110 ml konz. Salpetersäure vorgelegt und auf 0°C bis 5°C gekühlt. Zu diesem Gemisch werden 90,0 g (0,25 mol) N-(2-Brom-4-fluor-phenyl)-N-ethoxycarbonyl-ethansulfonsäureamid gegeben. Man läßt den Ansatz auf 15°C erwärmen, rührt zwei Stunden nach und gibt die Reaktionslösung dann auf 1 kg Eis. Der ausfallende Feststoff wird abgesaugt und in 300 ml Essigsäureethylester aufgenommen. Die organische Phase wird mit 100 ml Wasser und dann mit 100 ml gesättigter Natriumbicarbonat-Lösung gewaschen und über Natriumsulfat getrocknet. Nach Entfernen des Lösungsmittels am Rotationsverdampfer erhält man 92.3 g N-(2-Brom-4-fluor-5-nitro-phenyl)-N-ethoxycarbonyl-ethansulfonsäureamid (88 % der Theorie), das laut HPLC 95,7 %ig ist.

### Vierte Umsetzungsstufe:

### Beispiel (VIII-1)

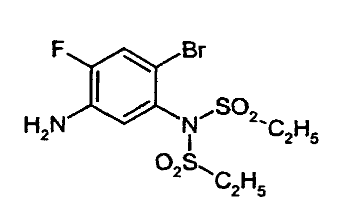

In einem 250 ml Dreihalskolben mit Rückflußkühler, Innenthermometer und Rührer werden 8,4 g (0,02 mol) N-(2-Brom-4-fluor-5-nitro-phenyl)-N-ethylsulfonyl-ethansulfonsäureamid unter Erwärmen in 100 ml Essigsäure gelöst und bei 35°C mit 6,7 g (0,12 mol) Eisenpulver versetzt. Man erhitzt vorsichtig bis die exotherme Reaktion in Gang kommt (ca. 40°C) und hält dann durch leichtes Kühlen mit einem Wasserbad die Temperatur bei ca. 40°C. Das Gemisch wird nach beendeter Reaktion noch eine Stunde nachgerührt und dann filtriert. Die abgetrennten Feststoffe werden mit 20 ml Essigsäure gewaschen und das Filtrat wird am Rotationsverdampfer eingeengt. Der Rückstand wird in 100 ml Essigsäureethylester aufgenommen, mit 50 ml Wasser und dann mit 50 ml gesättigter Natriumbicarbonat-Lösung gewaschen und über Natriumsulfat getrocknet. Nach Entfernen des Lösungsmittels am Rotationsverdampfer erhält man 2,9 g N-(2-Brom-4-fluor-5-amino-phenyl)-N-ethylsulfonyl-ethansulfonsäureamid (34 % der Theorie), das laut HPLC 91,0 %ig ist.

### Beispiel (VIII-2)

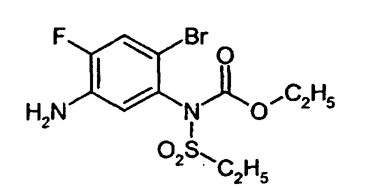

In einem 1 l Dreihalskolben mit Rückflußkühler, Innenthermometer und Rührer werden 40,0 g (0,1 mol) N-(2-Brom-4-fluor-5-nitro-phenyl)-N-ethoxycarbonyl-ethansulfonsäureamid unter Erwärmen in 400 ml Essigsäure gelöst und bei 20°C mit 33,0 g (0,6 mol) Eisenpulver versetzt. Man erhitzt vorsichtig bis die exotherme Reaktion in Gang kommt (ca. 50°C) und hält dann durch leichtes Kühlen mit einem Wasserbad die Temperatur bei ca. 50°C. Das Gemisch wird nach beendeter Reaktion noch eine Stunde nachgerührt und dann filtriert. Die abgetrennten Feststoffe werden mit 50 ml Essigsäure gewaschen und das Filtrat wird am Rotationsverdampfer eingeengt. Der Rückstand wird in 100 ml Essigsäureethylester und 100 ml Wasser aufgenommen, die Wasserphase wird neutralisiert (pH = 7-8) und die Phasen werden getrennt. Die org. Phase wird mit 100 ml Wasser und dann mit 100 ml gesättigter Natriumbicarbonat-Lösung gewaschen und über Natriumsulfat getrocknet. Nach Entfernen des Lösungsmittels am Rotationsverdampfer erhält man 34,3 g reines N-(2-Brom-4-fluor-5-amino-phenyl)-N-ethoxycarbonyl-ethansulfonsäureamid (93 % der Theorie).

### Fünfte Umsetzungsstufe:

### Beispiel (X-1)

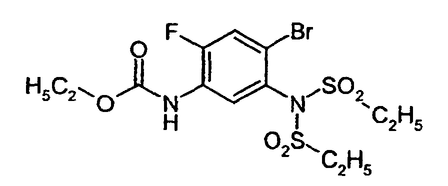

In einem 100 ml Dreihalskolben mit Innenthermometer, Tropftrichter und Rührer werden 2,8 g (0,0072 mol) N-(2-Brom-4-fluor-5-amino-phenyl)-N-ethylsulfonylethansulfonsäureamid in 50 ml Acetonitril und 0,8 g (0,01 mol) Pyridin gelöst und bei 5-10°C mit 0,9 g (0,008 mol) Chlorameisensäure-ethylester versetzt. Die Lösung wird 60 Minuten bei 20°C nachgerührt und dann auf 100 ml Eiswasser gegeben. Der ausfallende Feststoff wird abgesaugt, mit Wasser gewaschen und getrocknet. Man erhält 3,3 g N-(2-Brom-4-fluor-5-ethoxycarbonylamino-phenyl)-N-ethylsulfonylethansulfonsäureamid (99 % der Theorie) als hellbraunen Feststoff, das laut GC 90,6 %ig ist.

### Beispiel (X-2)

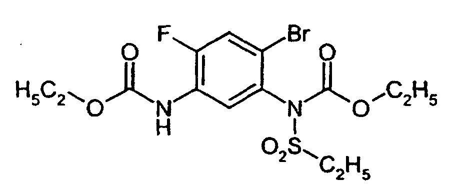

In einem 250 ml Dreihalskolben mit Innenthermometer, Tropftrichter und Rührer werden 16,6 g (0,045 mol) N-(2-Brom-4-fluor-5-amino-phenyl)-N-ethoxycarbonylethansulfonsäureamid in 120 ml Chloroform und 4,4 g (0,055 mol) Pyridin gelöst und bei 5°C bis 10°C mit 4,9 g (0,045 mol) Chlorameisensäure-ethylester versetzt. Die Lösung wird 1 h bei ca. 20°C nachgerührt und dann mit 100 ml Wasser versetzt. Die Phasen werden getrennt und die wäßrige Phase wird mit 50 ml Methylenchlorid extrahiert. Die vereinigten organischen Phasen werden mit 100 ml Wasser gewaschen und über Natriumsulfat getrocknet. Nach Entfernen des Lösungsmittels am Rotationsverdampfer erhält man 17,4 g N-(2-Brom-4-fluor-5-ethoxycarbonylaminophenyl)-N-ethoxycarbonyl-ethansulfonsäureamid (87 % der Theorie), das laut HPLC 99,0 %ig ist (Schmelzpunkt: 142-143°C).

### Sechste Umsetzungsstufe:

### Beispiel (XI-1)

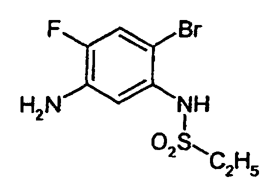

In einem 500 ml Dreihalskolben mit Innenthermometer und Rührer werden 18,5 g (0,05 mol) N-(2-Brom-4-fluor-5-amino-phenyl)-N-ethoxycarbonyl-ethansulfonsäureamid in 100 ml Wasser und 20 ml konz. Natronlauge gelöst und 4 h auf 60°C bis 70°C erwärmt. Man läßt auf Raumtemperatur abkühlen, säuert vorsichtig mit 2N-Salzsäure an (pH 1) und saugt den ausfallenden Feststoff ab. Nach Waschen mit Wasser trocknet man den Feststoff im Exsikkator und erhält 13,7 g reines N-(2-Brom-4-fluor-5-amino-phenyl)-ethansulfonsäureamid (92 % der Theorie).

### Beispiel (XI-2)

In einem 50 ml Dreihalskolben mit Innenthermometer und Rührer werden 0,2 g (0,005 mol) Natriumhydrid (60 %ig) in 20 ml N,N-Dimethyl-formamid vorgelegt und unter Kühlung (0°C bis 5°C) mit 2,15 g (0.0046 mol) N-(2-Brom-4-fluor-5-ethoxycarbonylamino-phenyl)-N-ethylsulfonyl-ethansulfonsäureamid versetzt. Die Lösung wird 60 Minuten im Eisbad nachgerührt und dann auf ein Gemisch aus 10 ml konz. Salzsäure und 50 ml Eiswasser gegeben. Der ausfallende Feststoff wird abgesaugt, mit Wasser gewaschen und getrocknet. Man erhält 1,4 g N-(2-Brom-4-fluor-5-etboxycarbonylamino-phenyl)-ethansulfonsäureamid (82 % der Theorie), das laut HPLC 78,6 %ig ist.

### Siebente Umsetzungsstufe:

### Beispiel (I-1)

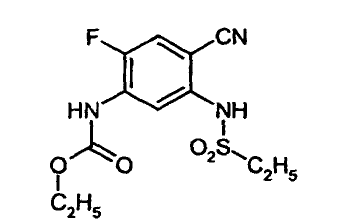

In einem 2 l Dreihalskolben mit Innenthermometer, Rückflußkühler und Rührer werden 383,8 g (1,00 mol) N-(2-Brom-4-fluor-5-ethoxycarbonylamino-phenyl)-ethansulfonsäureamid und 107,5 g (1,20 mol) Kupfer(I)-cyanid in 1 Liter trockenem N-Methyl-pyrrolidon gelöst und 6 Stunden auf 140°C erhitzt. Man läßt aufRaumtemperatur abkühlen und destilliert im Ölpumpenvakuum 800 ml N-Methyl-pyrrolidon ab. Der Rückstand wird mit 1 Liter Essigsäureethylester und salzsaurer Eisen(III)-chlorid-Lösung versetzt. Das zweiphasige System wird über Kieselgel filtriert und die Phasen werden getrennt. Die organische Phase wird über Natriumsulfat getrocknet und am Rotationsverdampfer eingeengt. Der klebrige Rückstand wird zum Waschen mit Petrolether verrührt, abgesaugt und getrocknet. Man erhält 275,9 g N-(2-Cyano-4-fluor-5-ethoxycarbonylamino-phenyl)-ethansulfonsäureamid (75 % der Theorie), das laut HPLC 86,0 %ig ist.

### Beispiel (I-2)

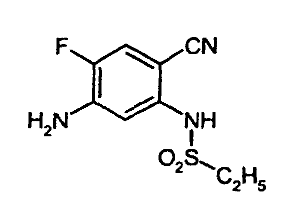

In einem 50 ml Zweihalskolben mit Innenthermometer, Rückflußkühler und Rührer werden 1,49 g (0,005 mol) N-(2-Brom-4-fluor-5-amino-phenyl)-ethansulfonsäureamid und 0,49 g (0,0055 mol) Kupfer(I)-cyanid in 10 ml trockenem N-Methyl-pyrrolidon gelöst und 6 Stunden auf 130°C erhitzt. Man läßt auf Raumtemperatur abkühlen und destilliert im Ölpumpenvakuum das N-Methyl-pyrrolidon ab. Im Rohprodukt befinden sich laut HPLC 76 % N-(2-Cyano-4-fluor-5-amino-phenyl)-ethansulfonsäureamid und 8,5 % Ausgangsverbindung.

## Patentansprüche

1. Verfahren zum Herstellen von N-substituierten 2,4-Diamino-5-fluor-benzonitrilen der allgemeinen Formel (I) 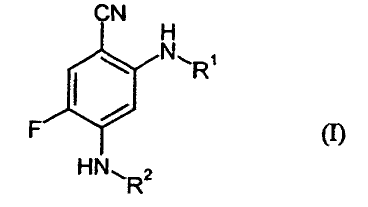 in welcher
R¹ für jeweils gegebenenfalls substituiertes Alkylsulfonyl oder Arylsulfonyl steht und
R² für Wasserstoff oder jeweils gegebenenfalls substituiertes Alkoxycarbonyl oder Aryloxycarbonyl steht,
**dadurch gekennzeichnet, dass** man in einer ersten Umsetzungsstufe 2-Brom-4-fluor-anilin der Formel (II) 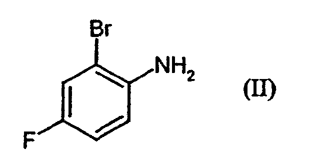 mit einem Acylierungs- oder Sulfonylierungsmittel der allgemeinen Formel (III)
X¹-R¹ (III)
in welcher
R¹ die oben angegebene Bedeutung hat und
X¹ für Halogen steht,
gegebenenfalls in Gegenwart eines oder mehrerer Reaktionshilfsmittel und gegebenenfalls in Gegenwart eines oder mehrerer Verdünnungsmittel bei Temperaturen zwischen -20°C und +100°C umsetzt,
die hierbei erhaltenen N-substituierten 2-Brom-4-fluor-aniline der allgemeinen Formel (IV) 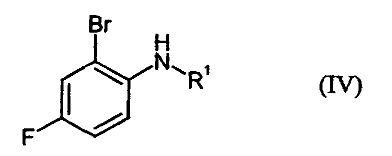 in welcher
R¹ die oben angegebene Bedeutung hat,
in einer zweiten Umsetzungsstufe mit Acylierungs- oder Sulfonylierungsmitteln der allgemeinen Formel (V)
X²-R³ (V)
in welcher
R³ für jeweils gegebenenfalls substituiertes Alkylsulfonyl, Alkylcarbonyl, Alkoxycarbonyl, Arylsulfonyl, Arylcarbonyl oder Aryloxycarbonyl steht und
X² für Halogen steht,
gegebenenfalls in Gegenwart eines oder mehrerer Reaktionshilfsmittel und gegebenenfalls in Gegenwart eines oder mehrerer Verdünnungsmittel bei Temperaturen zwischen -20°C und 100°C umsetzt,
die hierbei erhaltenen N-substituierten 2-Brom-4-fluor-aniline der allgemeinen Formel (VI) 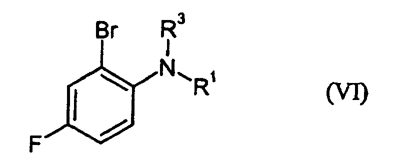 in welcher
R¹ und R³ die oben angegebene Bedeutung hat,
in einer dritten Umsetzungsstufe mit Nitrierungsmitteln, gegebenenfalls in Gegenwart eines oder mehrerer Nitrierungshilfsmittel und/oder gegebenenfalls in Gegenwart eines oder mehrerer Verdünnungsmittel bei Temperaturen zwischen -30°C und +50°C umsetzt,
die hierbei erhaltenen N-substituierten 2-Brom-4-fluor-5-nitro-aniline der allgemeinen Formel (VII) 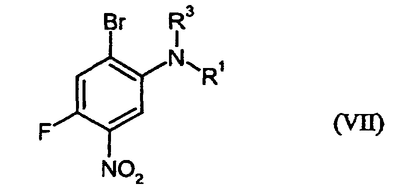 in welcher
R¹ und R³ die oben angegebene Bedeutung haben,
in einer vierten Umsetzungsstufe mit Reduktionsmitteln, gegebenenfalls in Gegenwart eines oder mehrerer Reduktionshilfsmittel und gegebenenfalls in Gegenwart eines oder mehrerer Verdünnungsmittel, bei Temperaturen zwischen 0°C und 150°C umsetzt,
die hierbei erhaltenen N-substituierten 4-Brom-6-fluor-1,3-phenylendiamine der allgemeinen Formel (VIII) 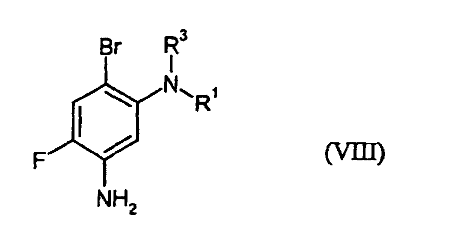 in welcher
R¹ und R³ die oben angegebene Bedeutung haben,
gegebenenfalls in einer fünften Umsetzungsstufe mit Acylierungsmitteln der allgemeinen Formel (IX)
X³-R²⁻¹ (IX)
in welcher
R²⁻¹ mit Ausnahme von Wasserstoff die oben für R² angegebene Bedeutung hat und
X³ für Halogen steht,
gegebenenfalls in Gegenwart eines oder mehrerer Reaktionshilfsmittel und gegebenenfalls in Gegenwart eines oder mehrerer Verdünnungsmittel bei Temperaturen zwischen -20°C und +100°C umsetzt,
die in der vierten bzw. fünften Stufe erhaltenen N-substituierten 4-Brom-6-fluor-1,3-phenylendiamine der allgemeinen Formel (X) 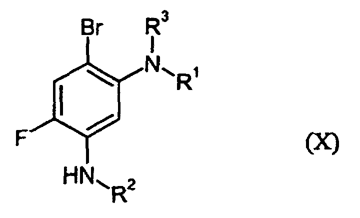 in welcher
R¹, R² und R³ die oben angegebene Bedeutung haben,
in einer sechsten Stufe mit Wasser in Gegenwart eines oder mehrerer Hydrolysehilfsmittel und gegebenenfalls in Gegenwart eines oder mehrerer Verdünnungsmittel bei Temperaturen zwischen 0°C und 120°C umsetzt,
und die hierbei erhaltenen N-substituierten 4-Brom-6-fluor-1,3-phenylendiamine der allgemeinen Formel (XI) 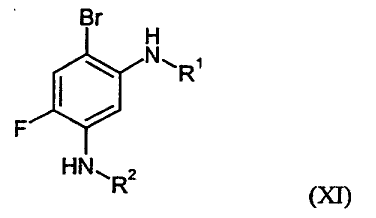 in welcher
R¹ und R² die oben angegebene Bedeutung haben,
in einer siebenten Umsetzungsstufe mit einem Metallcyanid gegebenenfalls in Gegenwart eines oder mehrerer Verdünnungsmittel bei Temperaturen zwischen 50°C und 200°C umsetzt.

2. Verfahren gemäß Anspruch 1, **dadurch gekennzeichnet, dass**
R¹ für gegebenenfalls durch Fluor, Chlor oder Brom substituiertes Alkylsulfonyl mit 1 bis 6 Kohlenstoffatomen oder für gegebenenfalls durch Fluor, Chlor, Brom, C₁-C₄-Alkyl oder C₁-C₄-Alkoxy substituiertes Phenylsulfonyl steht,
R² für Wasserstoff, für gegebenenfalls durch Fluor, Chlor oder Brom substituiertes Alkoxycarbonyl mit 1 bis 6 Kohlenstoffatomen in der Alkylgruppe, oder für gegebenenfalls durch Fluor, Chlor, Brom, C₁-C₄-Alkyl oder C₁-C₄-Alkoxy substituiertes Phenoxycarbonyl steht,
R³ für jeweils gegebenenfalls durch Fluor, Chlor oder Brom substituiertes Alkylsulfonyl, Alkylcarbonyl oder Alkoxycarbonyl mit jeweils 1 bis 6 Kohlenstoffatomen in den Alkylgruppen, oder für jeweils gegebenenfalls durch Fluor, Chlor, Brom, C₁-C₄-Alkyl oder C₁-C₄-Alkoxy substituiertes Phenylsulfonyl, Phenylcarbonyl oder Phenoxycarbonyl steht,
X¹ für Fluor, Chlor, Brom oder Iod steht,
X² für Fluor, Chlor, Brom oder Iod steht, und
X³ für Fluor, Chlor, Brom oder Iod steht.

3. Verfahren gemäß Anspruch 1, **dadurch gekennzeichnet, dass**
R¹ für jeweils gegebenenfalls durch Fluor oder Chlor substituiertes Methylsulfonyl, Ethylsulfonyl, n- oder i-Propylsulfonyl, n-, i-, s- oder t-Butylsulfonyl oder für jeweils gegebenenfalls durch Fluor, Chlor, Brom, Methyl, Ethyl, n-oder i-Propyl, n-, i-, s- oder t-Butyl, Methoxy, Ethoxy, n- oder i-Propoxy substituiertes Phenylsulfonyl steht,
R² für Wasserstoff, für jeweils gegebenenfalls durch Fluor oder Chlor substituiertes Methoxycarbonyl, Ethoxycarbonyl, n- oder i-Propoxycarbonyl, n-, i-, s- oder t-Butoxycarbonyl, oder für gegebenenfalls durch Fluor, Chlor, Brom, Methyl, Ethyl, n- oder i-Propyl, n-, i-, s- oder t-Butyl, Methoxy, Ethoxy, n- oder i-Propoxy substituiertes Phenoxycarbonyl steht,
R³ für jeweils gegebenenfalls durch Fluor oder Chlor substituiertes Methylsulfonyl, Ethylsulfonyl, n- oder i-Propylsulfonyl, n-, i-, s- oder t-Butylsulfonyl, Methylcarbonyl, Ethylcarbonyl, n- oder i-Propylcarbonyl, n-, i-, s- oder t-Butylcarbonyl, Methoxycarbonyl, Ethoxycarbonyl, n- oder i-Propoxycarbonyl, oder für jeweils gegebenenfalls durch Fluor, Chlor, Brom, Methyl, Ethyl, n- oder i-Propyl, n-, i-, s- oder t-Butyl, Methoxy, Ethoxy, n- oder i-Propoxy substituiertes Phenylsulfonyl, Phenylcarbonyl oder Phenoxycarbonyl steht,
X¹ für Fluor, Chlor oder Brom steht,
X² für Fluor, Chlor oder Brom steht, und
X³ für Fluor, Chlor oder Brom steht.

4. Verfahren gemäß Anspruch 1, **dadurch gekennzeichnet, dass**
R¹ für jeweils gegebenenfalls durch Fluor oder Chlor substituiertes Methylsulfonyl, Ethylsulfonyl, n- oder i-Propylsulfonyl, n- oder i-Butylsulfonyl steht,
R² für Wasserstoff oder für Methoxycarbonyl, Ethoxycarbonyl, n- oder i-Propoxycarbonyl steht,
R³ für jeweils gegebenenfalls durch Fluor oder Chlor substituiertes Methylsulfonyl, Ethylsulfonyl, n- oder i-Propylsulfonyl, n- oder i-Butylsulfonyl, Methylcarbonyl, Ethylcarbonyl, n- oder i-Propylcarbonyl, n-, i-, s- oder t-Butylcarbonyl, für Methoxycarbonyl, Ethoxycarbonyl, n- oder i-Propoxycarbonyl steht,
X¹ für Chlor steht,
X² für Chlor steht, und
X³ für Chlor steht.

5. N-substituierte 2-Brom-4-fluor-aniline der allgemeinen Formel (IV) 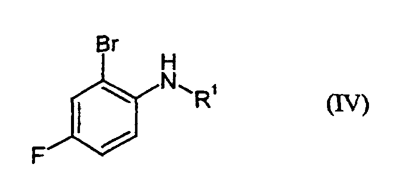 in welcher
R¹ die in einem der Ansprüche 1 bis 4 angegebene Bedeutung hat,
ausgenommen die Verbindungen N-(2-Brom-4-fluor-phenyl)-benzosulfonamid und N-(2-Brom-4-fluor-phenyl)-1,1,1-trifluor-methansulfonamid.

6. N-substituierte 2-Brom-4-fluor-aniline der allgemeinen Formel 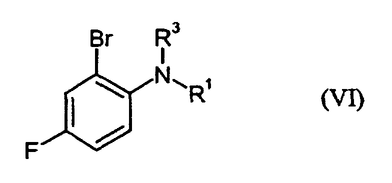 in welcher
R¹ und R³ die in einem der Ansprüche 1 bis 4 angegebene Bedeutung haben.

7. N-substituierte 2-Brom-4-fluor-5-nitro-aniline der allgemeinen Formel (VII) 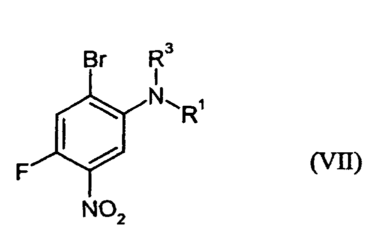 in welcher
R¹ und R³ die in einem der Ansprüche 1 bis 4 angegebene Bedeutung haben.

8. N-substituierte 4-Brom-6-fluor-1,3-phenylendiamine der allgemeinen Formel (VIII) 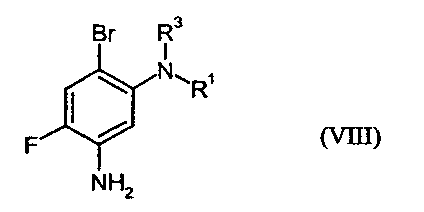 in welcher
R¹ und R³ die in einem der Ansprüche 1 bis 4 angegebene Bedeutung haben.

9. N-substituierte 4-Brom-6-fluor-1,3-phenylendiamine der allgemeinen Formel (XI) 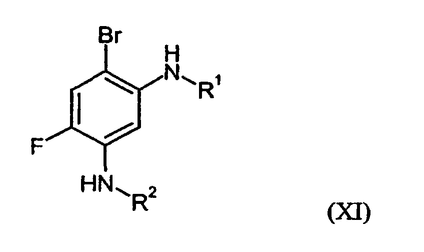 in welcher
R¹ und R² die in einem der Ansprüche 1 bis 4 angegebene Bedeutung haben.

## Claims

1. Process for preparing N-substituted 2,4-diamino-5-fluoro-benzonitriles of the general formula (I) 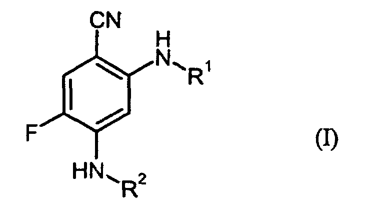 in which
R¹ represents in each case optionally substituted alkylsulphonyl or arylsulphonyl and
R² represents hydrogen or in each case optionally substituted alkoxycarbonyl or aryloxycarbonyl,
**characterized in that**, in a first reaction step, 2-bromo-4-fluoro-aniline of the formula (II) 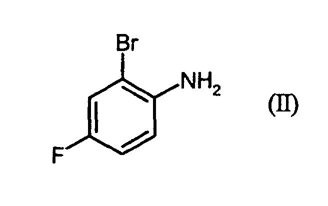 is reacted with an acylating or sulphonylating agent of the general formula (III)
X¹-R¹ (III)
in which
R¹ is as defined above and
X¹ represents halogen,
if appropriate in the presence of one or more reaction auxiliaries and if appropriate in the presence of one or more diluents, at temperatures between -20°C and +100°C,
the resulting N-substituted 2-bromo-4-fluoro-anilines of the general formula (IV) 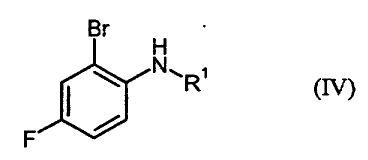 in which
R¹ is as defined above,
are, in a second reaction step, reacted with acylating or sulphonylating agents of the general formula (V)
X²-R³ (V)
in which
R³ represents in each case optionally substituted alkylsulphonyl, alkylcarbonyl, alkoxycarbonyl, arylsulphonyl, arylcarbonyl or aryloxycarbonyl and
X² represents halogen,
if appropriate in the presence of one or more reaction auxiliaries and if appropriate in the presence of one or more diluents, at temperatures between -20°C and 100°C,
the resulting N-substituted 2-bromo-4-fluoro-anilines of the general formula (VI) 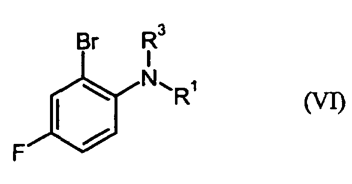 in which
R¹ and R³ are each as defined above,
are reacted, in a third reaction step, with nitrating agents, if appropriate in the presence of one or more nitration auxiliaries and/or if appropriate in the presence of one or more diluents, at temperatures between -30°C and +50°C,
the resulting N-substituted 2-bromo-4-fluoro-5-nitro-anilines of the general formula (VII) 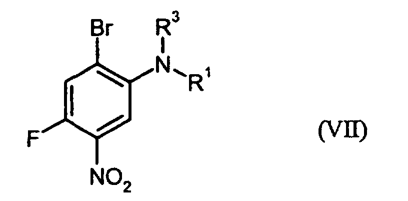 in which
R¹ and R³ are each as defined above,
are, in a fourth reaction step, reacted with reducing agents, if appropriate in the presence of one or more reduction auxiliaries and if appropriate in the presence of one or more diluents, at temperatures between 0°C and 150°C,
the resulting N-substituted 4-bromo-6-fluoro-1,3-phenylenediamines of the general formula (VIII) 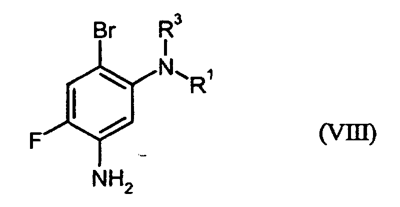 in which
R¹ and R³ are each as defined above,
are, if appropriate, reacted, in a fifth reaction step, with acylating agents of the general formula (IX)
X³-R²⁻¹ (IX)
in which
R²⁻¹ has the meanings given above for R², except for hydrogen, and
X³ represents halogen,
if appropriate in the presence of one or more reaction auxiliaries and if appropriate in the presence of one or more diluents, at temperatures between -20°C and +100°C,
the N-substituted 4-bromo-6-fluoro-1,3-phenylenediamines, obtained in the fourth or fifth step, of the general formula (X) 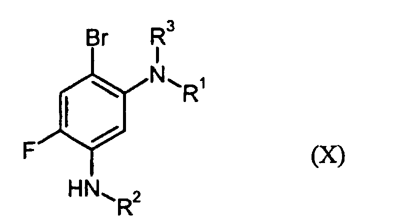 in which
R¹, R² and R³ are each as defined above,
are, in a sixth step, reacted with water in the presence of one or more hydrolysis auxiliaries and if appropriate in the presence of one or more diluents, at temperatures between 0°C and 120°C,
and the resulting N-substituted 4-bromo-6-fluoro-1,3-phenylenediamines of the general formula (XI) 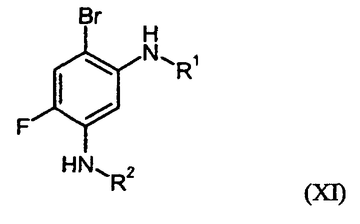 in which
R¹ and R² are each as defined above,
are, in a seventh reaction step, reacted with a metal cyanide, if appropriate in the presence of one or more diluents, at temperatures between 50°C and 200°C.

2. Process according to Claim 1, **characterized in that**
R¹ represents optionally fluorine-, chlorine- or bromine-substituted alkylsulphonyl having 1 to 6 carbon atoms or represents optionally fluorine-, chlorine-, bromine-, C₁-C₄-alkyl- or C₁-C₄-alkoxysubstituted phenylsulphonyl,
R² represents hydrogen, represents optionally fluorine-, chlorine- or bromine-substituted alkoxycarbonyl having 1 to 6 carbon atoms in the alkyl group, or represents optionally fluorine-, chlorine-, bromine-, C₁-C₄-alkyl- or C₁-C₄-alkoxy-substituted phenoxycarbonyl,
R³ represents in each case optionally fluorine-, chlorine- or bromine-substituted alkylsulphonyl, alkylcarbonyl or alkoxycarbonyl having in each case 1 to 6 carbon atoms in the alkyl groups, or represents in each case optionally fluorine-, chlorine-, bromine-, C₁-C₄-alkyl- or C₁-C₄-alkoxy-substituted phenylsulphonyl, phenylcarbonyl or phenoxycarbonyl,
X¹ represents fluorine, chlorine, bromine or iodine,
X² represents fluorine, chlorine, bromine or iodine, and
X³ represents fluorine, chlorine, bromine or iodine.

3. Process according to Claim 1, **characterized in that**
R¹ represents in each case optionally fluorine- or chlorine-substituted methylsulphonyl, ethylsulphonyl, n- or i-propylsulphonyl, n-, i-, s- or t-butylsulphonyl or represents in each case optionally fluorine-, chlorine-, bromine-, methyl-, ethyl-, n- or i-propyl-, n-, i-, s- or t-butyl-, methoxy-, ethoxy-, n- or i-propoxy-substituted phenylsulphonyl,
R² represents hydrogen, represents in each case optionally fluorine- or chlorine-substituted methoxycarbonyl, ethoxycarbonyl, n- or i-propoxycarbonyl, n-, i-, s- or t-butoxycarbonyl, or represents optionally fluorine-, chlorine-, bromine-, methyl-, ethyl-, n- or i-propyl-, n-, i-, s- or t-butyl-, methoxy-, ethoxy-, n- or i-propoxy-substituted phenoxycarbonyl,
R³ represents in each case optionally fluorine- or chlorine-substituted methylsulphonyl, ethylsulphonyl, n- or i-propylsulphonyl, n-, i-, s- or t-butylsulphonyl, methylcarbonyl, ethylcarbonyl, n- or i-propylcarbonyl, n-, i-, s- or t-butylcarbonyl, methoxycarbonyl, ethoxycarbonyl, n- or i-propoxycarbonyl, or represents in each case optionally fluorine-, chlorine-, bromine-, methyl-, ethyl-, n- or i-propyl-, n-, i-, s- or t-butyl-, methoxy-, ethoxy-, n- or i-propoxy-substituted phenylsulphonyl, phenylcarbonyl or phenoxycarbonyl,
X¹ represents fluorine, chlorine or bromine,
X² represents fluorine, chlorine or bromine, and
X³ represents fluorine, chlorine or bromine.

4. Process according to Claim 1, **characterized in that**
R¹ represents in each case optionally fluorine- or chlorine-substituted methylsulphonyl, ethylsulphonyl, n- or i-propylsulphonyl, n- or i-butylsulphonyl,
R² represents hydrogen or represents methoxycarbonyl, ethoxycarbonyl, n- or i-propoxycarbonyl,
R³ represents in each case optionally fluorine- or chlorine-substituted methylsulphonyl, ethylsulphonyl, n- or i-propylsulphonyl, n- or i-butylsulphonyl, methylcarbonyl, ethylcarbonyl, n- or i-propylcarbonyl, n-, i-, s- or t-butylcarbonyl, represents methoxycarbonyl, ethoxycarbonyl, n- or i-propoxycarbonyl,
X¹ represents chlorine,
X² represents chlorine, and
X³ represents chlorine.

5. N-substituted 2-bromo-4-fluoro-anilines of the general formula (IV) 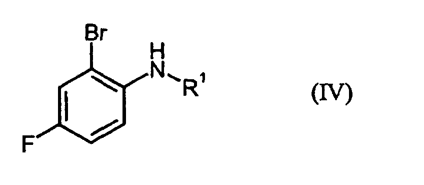 in which
R¹ is as defined in any of Claims 1 to 4,
except for the compounds N-(2-bromo-4-fluoro-phenyl)-benzenesulphonamide and N-(2-bromo-4-fluoro-phenyl)-1,1,1-trifluoromethanesulphonamide.

6. N-substituted 2-bromo-4-fluoro-anilines of the general formula 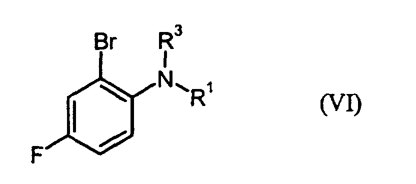 in which
R¹ and R³ are each as defined in any of Claims 1 to 4.

7. N-substituted 2-bromo-4-fluoro-5-nitro-anilines of the general formula (VII) 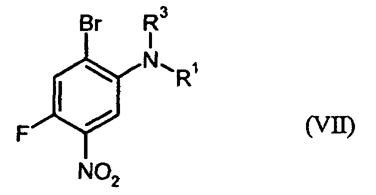 in which
R¹ and R³ are each as defined in any of Claims 1 to 4.

8. N-substituted 4-bromo-6-fluoro-1,3-phenylenediamines of the general formula (VIII) 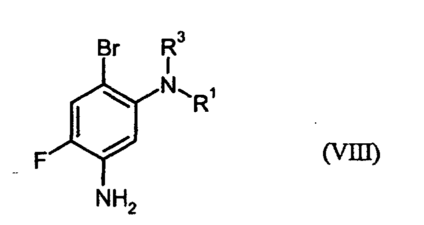 in which
R¹ and R³ are each as defined in any of Claims 1 to 4.

9. N-substituted 4-bromo-6-fluoro-1,3-phenylenediamines of the general formula (XI) 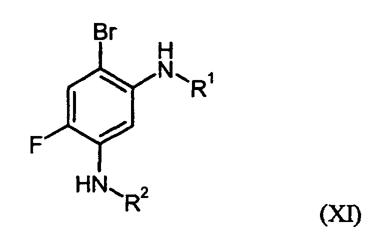 in which
R¹ and R² are each as defined in any of Claims 1 to 4.

## Revendications

1. Procédé de production de 2,4-diamino-5-fluorobenzonitriles N-substitués, de formule générale (I) 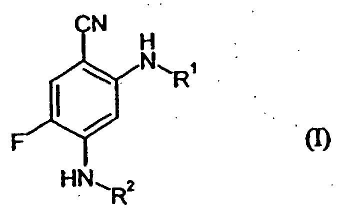 dans laquelle
R¹ est un groupe alkylsulfonyle ou arylsulfonyle chacun éventuellement substitué et
R² représente l'hydrogène ou un groupe alkoxycarbonyle ou aryloxycarbonyle chacun éventuellement substitué,
**caractérisé en ce que**, dans une première étape de réaction, on fait réagir la 2-bromo-4-fluoraniline de formule (II) 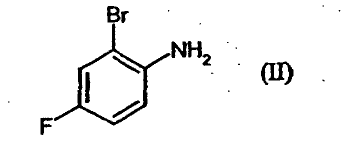 avec un agent d'acylation ou de sulfonylation de formule générale (III)
X¹ - R¹ (III)
dans laquelle
R¹ a la définition indiquée ci-dessus et
X¹ est un halogène,
éventuellement en présence d'un ou plusieurs auxiliaires de réaction et, le cas échéant, en présence d'un ou plusieurs diluants, à des températures comprises entre -20°C et +100°C,
dans une deuxième étape de réaction, on fait réagir les 2-bromo-4-fluoranilines N-substituées ainsi obtenues, de formule générale (IV) 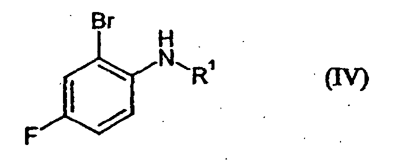 dans laquelle
R¹ a la définition indiquée ci-dessus,
avec des agents d'acylation ou de sulfonylation de formule générale (V)
X² - R³ (V)
dans laquelle
R³ est un groupe alkylsulfonyle, alkylcarbonyle, alkoxycarbonyle, arylsulfonyle, arylcarbonyle ou aryloxycarbonyle chacun éventuellement substitué et
X² est un halogène,
éventuellement en présence d'un ou plusieurs auxiliaires de réaction et, le cas échéant, en présence d'un ou plusieurs diluants, à des températures comprises entre -20°C et +100°C,
dans une troisième étape de réaction,
on fait réagir les 2-bromo-4-fluoranilines N-substituées ainsi obtenues de formule générale (VI) 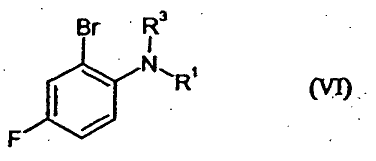 dans laquelle
R¹ et R³ ont la définition indiquée ci-dessus,
avec des agents de nitration, éventuellement en présence d'un ou plusieurs auxiliaires de nitration et/ou, le cas échéant, en présence d'un ou plusieurs diluants, à des températures comprises entre -30°C et +50°C, dans une quatrième étape de réaction,
on fait réagir les 2-bromo-4-fluoro-5-nitroanilines N-substituées ainsi obtenues, de formule générale (VII) 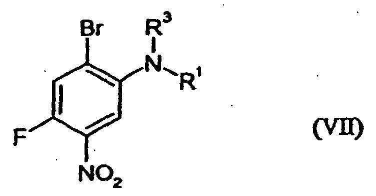 dans laquelle
R¹ et R³ ont la définition indiquée ci-dessus,
avec des agents réducteurs, éventuellement en présence d'un ou plusieurs auxiliaires de réduction et, le cas échéant, en présence d'un ou plusieurs diluants, à des températures comprises entre 0°C et 150°C,
le cas échéant dans une cinquième étape de réaction, on fait réagir les 4-bromo-6-fluoro-1,3-phénylène-diamines N-substituées ainsi obtenues, de formule générale (VIII) 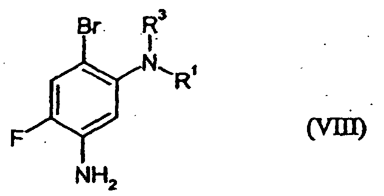 dans laquelle
R¹ et R³ ont la définition indiquée ci-dessus,
avec des agents d'acylation de formule générale (IX)
X³ - R²⁻¹ (IX)
dans laquelle
R²⁻¹ a la définition indiquée ci-dessus pour R² à l'exception de l'hydrogène et
X³ est un halogène,
éventuellement en présence d'un ou plusieurs auxiliaires de réaction et, le cas échéant, en présence d'un ou plusieurs diluants, à des températures comprises entre -20°C et +100°C,
dans une sixième étape,
on fait réagir les 4-bromo-6-fluoro-1,3-phénylène-diamines N-substituées obtenues dans la quatrième et dans la cinquième étape, de formule générale (X) 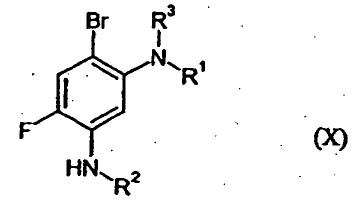 dans laquelle
R¹, R² et R³ ont la définition indiquée ci-dessus,
avec l'eau en présence d'un ou plusieurs auxiliaires d'hydrolyse et, le cas échéant, en présence d'un ou plusieurs diluants, à des températures comprises entre 0°C et 120°C,
et dans une septième étape de réaction,
on fait réagir les 4-bromo-6-fluoro-1,3-phénylène-diamines N-substituées ainsi obtenues, de formule générale (XI) 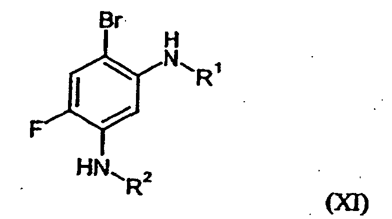 dans laquelle
R¹ et R² ont la définition indiquée ci-dessus,
avec un cyanure métallique, éventuellement en présence d'un ou plusieurs diluants, à des températures comprises entre 50°C et 200°C.

2. Procédé suivant la revendication 1, **caractérisé en ce que**
R¹ est un reste alkylsulfonyle ayant 1 à 6 atomes de carbone éventuellement substitué par du fluor, du chlore ou du brome, ou un reste phénylsulfonyle éventuellement substitué par du fluor, du chlore, du brome, un radical alkyle en C₁ à C₄ ou alkoxy en C₁ à C₄,
R² représente l'hydrogène, un reste alkoxycarbonyle ayant 1 à 6 atomes de carbone dans le groupe alkyle, éventuellement substitué par du fluor, du chlore ou du brome, ou un groupe phénoxycarbonyle éventuellement substitué par du fluor, du chlore, du brome, un radical alkyle en C₁ à C₄ ou alkoxy en C₁ à C₄,
R³ est un reste alkylsulfonyle, alkylcarbonyle ou alkoxycarbonyle ayant dans chaque cas 1 à 6 atomes de carbone dans les groupes alkyle et chacun étant éventuellement substitué par du fluor, du chlore ou du brome, ou un reste phénylsulfonyle, phénylcarbonyle ou phénoxy-carbonyle dont chacun est éventuellement substitué par du fluor, du chlore, du brome, un radical alkyle en C₁ à C₄ ou alkoxy en C₁ à C₄,
X¹ représente le fluor, le chlore, le brome ou l'iode,
X² représente le fluor, le chlore, le brome ou l'iode et
X³ représente le fluor, le chlore, le brome ou l'iode.

3. Procédé suivant la revendication 1, **caractérisé en ce que**
R¹ est un reste méthylsulfonyle, éthylsulfonyle, n-propylsulfonyle, isopropylsulfonyle, n-butylsulfonyle, isobutylsulfonyle, sec.-butylsulfonyle ou tertiobutylsulfonyle, chacun éventuellement substitué par du fluor ou du chlore, ou un reste phénylsulfonyle éventuellement substitué par du fluor, du chlore, du brome, un radical méthyle, éthyle, n-propyle, isopropyle, n-butyle, isobutyle, sec.-butyle, tertiobutyle, méthoxy, éthoxy, n-propoxy ou isopropoxy,
R² représente l'hydrogène, un reste méthoxycarbonyle, éthoxycarbonyle, n-propoxycarbonyle, isopropoxycarbonyle, n-butoxycarbonyle, isobutoxycarbonyle, sec.-butoxycarbonyle ou tertiobutoxycarbonyle, chacun éventuellement substitué par du fluor ou du chlore, ou un reste phénoxycarbonyle éventuellement substitué par du fluor, du chlore, du brome, un radical méthyle, éthyle, n-propyle, isopropyle, n-butyle, isobutyle, sec.-butyle, tertiobutyle, méthoxy, éthoxy, n-propoxy ou isopropoxy,
R³ est un reste méthylsulfonyle, éthylsulfonyle, n-propylsulfonyle, isopropylsulfonyle, n-butylsulfonyle, isobutylsulfonyle, sec.-butylsulfonyle, tertiobutylsulfonyle, méthylcarbonyle, éthylcarbonyle, n-propylcarbonyle, isopropylcarbonyle, n-butylcarbonyle, isobutylcarbonyle, sec.-butylcarbonyle, tertiobutylcarbonyle, méthoxycarbonyle, éthoxycarbonyle, n-propoxycarbonyle ou isopropoxycarbonyle, chacun éventuellement substitué par du fluor ou du chlore, ou un reste phénylsulfonyle, phénylcarbonyle ou phénoxycarbonyle, chacun éventuellement substitué par du fluor, du chlore, du brome, un radical méthyle, éthyle, n-propyle, isopropyle, n-butyle, isobutyle, sec.-butyle, tertiobutyle, méthoxy, éthoxy, n-propoxy ou isopropoxy,
X¹ représente le fluor, le chlore ou le brome,
X² représente le fluor, le chlore ou le brome et
X³ représente le fluor, le chlore ou le brome.

4. Procédé suivant la revendication 1, **caractérisé en ce que**
R¹ est un reste méthylsulfonyle, éthylsulfonyle, n-propylsulfonyle, isopropylsulfonyle, n-butylsulfonyle ou isobutylsulfonyle, chacun éventuellement substitué par du fluor ou du chlore,
R² est l'hydrogène ou un reste méthoxycarbonyle, éthoxycarbonyle, n-propoxycarbonyle ou isopropoxycarbonyle,
R³ est un reste méthylsulfonyle, éthylsulfonyle, n-propylsulfonyle, isopropylsulfonyle, n-butylsulfonyle, isobutylsulfonyle, méthylcarbonyle, éthylcarbonyle, n-propylcarbonyle, isopropylcarbonyle, n-butylcarbonyle, isobutylcarbonyle, sec.- butylcarbonyle ou tertiobutylcarbonyle, chacun éventuellement substitué par du fluor ou du chlore, un reste méthoxycarbonyle, éthoxycarbonyle, n-propoxycarbonyle ou isopropoxycarbonyle,
X¹ représente le chlore,
X² représente le chlore et
X³ représente le chlore.

5. 2-bromo-4-fluoranilines N-substituées de formule générale (IV) 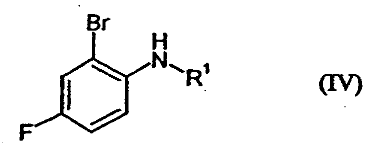 dans laquelle
R¹ a la définition indiquée dans l'une des revendications 1 à 4,
excepté les composés N-(2-bromo-4-fluorophényl)-benzosulfonamide et N-(2-bromo-4-fluorophényl)-1,1,1-trifluorométhanesulfonamide.

6. 2-bromo-4-fluoranilines N-substituées de formule générale 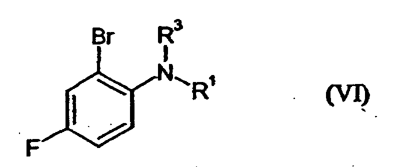 dans laquelle
R¹ et R³ ont la définition indiquée dans l'une des revendications 1 à 4.

7. 2-bromo-4-fluoro-5-nitro-anilines N-substituées de formule générale (VII) 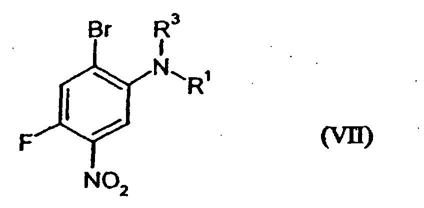 dans laquelle
R¹ et R³ ont la définition indiquée dans l'une des revendications 1 à 4.

8. 4-bromo-6-fluoro-1,3-phénylène-diamines N-substituées de formule générale (VIII) 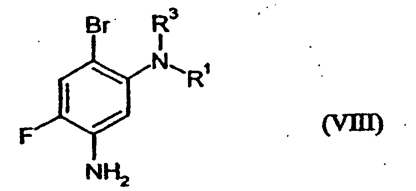 dans laquelle
R¹ et R³ ont la définition indiquée dans l'une des revendications 1 à 4.

9. 4-bromo-6-fluoro-1,3-phénylène-diamines N-substituées de formule générale (XI) 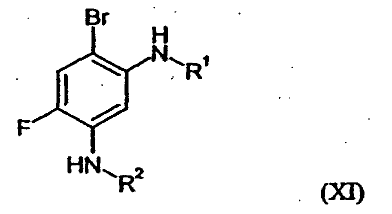 dans laquelle
R¹ et R² ont la définition indiquée dans l'une des revendications 1 à 4.
